# EUROPEAN PATENT SPECIFICATION

(11) **EP 2 193 802 B1**
(45) Date of publication and mention of the grant of the patent: **09.05.2012**
(21) Application number: 10151641.7
(22) Date of filing: 08.02.2006
(51) Int. Cl.: A61K 39/00, A61K 39/215, C07K 16/10

(54) **Neutralizing monoclonal antibodies against severe acute respiratory syndrome-associated coronavirus**
Neutralisierung monoklonaler Antikörper gegen mit schwerem akutem Respirationssyndrom assoziiertem Coronavirus
Anticorps monoclonaux neutralisant contre le coronavirus associé au syndrôme respiratoire aigu grave

(30) Priority: 08.02.2005 US 651046; 31.05.2005 US 141925
(43) Date of publication of application: 09.06.2010
(62) Divisional of application: 06720569.0
(73) Proprietor: The New York Blood Center, Inc., New York, New York 10021 (US)
(72) Inventor: Jiang, Shibo, New York, NY 11365 (US); He, Yuxian, Bronx, NY 10463 (US)
(74) Representative: Thomas, Simon

(56) References cited:
- HE Y ET AL: "Inactivated SARS-CoV vaccine elicits high titers of spike protein-specific antibodies that block receptor binding and virus entry" BIOCHEMICAL AND BIOPHYSICAL RESEARCH COMMUNICATIONS, ACADEMIC PRESS INC. ORLANDO, FL, US, vol. 325, no. 2, 10 December 2004 (2004-12-10), pages 445-452, XP004626814 ISSN: 0006-291X
- XIAO X ET AL: "The SARS-CoV S glycoprotein: expression and functional characterization" BIOCHEMICAL AND BIOPHYSICAL RESEARCH COMMUNICATIONS, ACADEMIC PRESS INC. ORLANDO, FL, US, vol. 312, no. 4, 26 December 2003 (2003-12-26), pages 1159-1164, XP004476398 ISSN: 0006-291X
- BABCOCK GREGORY J ET AL: "Amino acids 270 to 510 of the severe acute respiratory syndrome coronavirus spike protein are required for interaction with receptor" JOURNAL OF VIROLOGY, vol. 78, no. 9, May 2004 (2004-05), pages 4552-4560, XP002546670 ISSN: 0022-538X
- TRAGGIAI E ET AL: "An efficient method to make human monoclonal antibodies from memory B cells: potent neutralization of SARS coronavirus" NATURE MEDICINE, NATURE PUBLISHING GROUP, NEW YORK, NY, US, vol. 10, no. 8, 11 July 2004 (2004-07-11), pages 871-875, XP002385893 ISSN: 1078-8956
- HE YUXIAN ET AL: "Receptor-binding domain of SARS-CoV spike protein induces highly potent neutralizing antibodies: implication for developing subunit vaccine" BIOCHEMICAL AND BIOPHYSICAL RESEARCH COMMUNICATIONS, vol. 324, no. 2, 12 November 2004 (2004-11-12), pages 773-781, XP004599673 ISSN: 0006-291X
- SUI JIANHUA ET AL: "Potent neutralization of severe acute respiratory syndrome (SARS) coronavirus by a human mAb to S1 protein that blocks receptor association" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.0307140101, vol. 101, no. 8, 24 February 2004 (2004-02-24), pages 2536-2541, XP002332534 ISSN: 0027-8424
- HE YUXIAN ET AL: "receptor-binding domain of severe acute respiratory syndrome coronavirus spike protein contains multiple conformation-dependent epitopes that induce highly potent neutralizing antibodies" THE JOURNAL OF IMMUNOLOGY, vol. 174, 1 June 2005 (2005-06-01), pages 4908-4915, XP002589166 ISSN: 0022-1767

### SEQUENCE LISTING

<110> Jiang, Shibo
   He, Yuxian
<120> Neutralizing Monoclonal Antibodies Against Severe Acute Respiratory Syndrome associated Coronavirus
<130> 1958427-00011
<140> 11/351,108
   <141> 2006-02-28
<150> US 11/141,925
   <151> 2005-05-31
<150> US 60/651,046
   <151> 2005-02-28
<150> US 60/576,118
   <151> 2004-06-02
<160> 4
<170> PatentIn version 3.3
<210> 1
   <211> 17
   <212> PRT
   <213> Human coronavirus
<400> 1
   Asn Tyr Asn Tyr Lys Tyr Arg Tyr Leu Arg His Gly Lys Leu Arg Pro
   1               5                   10                  15
   Phe
<210> 2
   <211> 17
   <212> PRT
   <213> Human coronavirus
<400> 2
   Tyr Leu Arg His Gly Lys Leu Arg Pro Phe Glu Arg Asp Ile Ser Asn
   1               5                   10                  15
   Val
<210> 3
   <211> 17
   <212> PRT
   <213> Human coronavirus
<400> 3
   Arg Pro Phe Glu Arg Asp Ile Ser Asn Val Pro Phe Ser Pro Asp Gly
   1               5                   10                  15
   Lys
<210> 4
   <211> 10
   <212> PRT
   <213> Human coronavirus
<400> 4
   Arg Pro Phe Glu Arg Asp Ile Ser Asn Val
   1               5                   10

## Description

### BACKGROUND OF THE INVENTION

Severe acute respiratory syndrome (SARS) is a recently-recognized, febrile severe lower respiratory illness that is the result of an infection caused by a novel coronavirus (SARS-CoV) (1-5). The global outbreak of SARS was contained, but concerns remain over the possibility of future recurrences, especially with recent reports of laboratory-acquired infections (6). However, no effective treatment or prophylaxis is currently available to combat this deadly virus (7, 8).

Like other coronaviruses, SARS-CoV is an enveloped virus containing a large, positive-stranded RNA genome that encodes viral replicase proteins and structural proteins including spike (S), membrane (M), envelope (E), nucleocapsid (N), and several uncharacterized proteins (4, 5, 9). Phylogenetic analyses indicate that SARS-CoV is distinct from the three known antigenic groups of coronaviruses. Therefore, post-genomic characterization of SARS-CoV is important for developing anti-SARS therapeutics and vaccines (10, 11).

Coronavirus infection is initiated by attachment of the S protein to the specific host receptor, which triggers a conformational change in the S protein. The S protein of SARS-CoV is a types I transmembrane glycoprotein with a predicted length of 1,255 amino acids that contains a leader (residues 1-14), an ectodomain (residues 15-1190), a transmembrane domain (residues 1191-1227), and a short intracellular tail (residues 1227-1255) (5). Unlike many other coronaviruses, such as the mouse hepatitis virus (MHV)(12,13), in which the S protein is post-translationally cleaved into S1 and S2 subunits, no typical cleavage motif has been identified in the SARS-CoV S protein (5). Nonetheless, its S1 and S2 domains were predicted by sequence alignment with other coronavirus S proteins (5, 14). The S2 domain (residues 681-1255) of SARS-CoV S protein containing a putative fusion peptide and two heptad repeat (HR1 and HR2) regions is responsible for fusion between viral and target cell membranes. It has been found that the HR1 and HR2 regions can associate to form a six-helix bundle structure (15-18), resembling the fusion-active core of the HIV gp41 (19) and the MHV S protein (20,21). The S1 domain of SARS-CoV S protein mediates virus-binding with angiotensin-converting enzyme 2 (ACE2), the functional receptor for SARS-CoV on susceptible cells (22-25). Recently, a 193-amino-acid small fragment within S1 domain (residues 318-510) was identified as a receptor-binding domain (RBD), which is sufficient to associate with ACE2 (26-28).

The S proteins of coronaviruses are major antigenic determinants that induce the production of neutralizing antibodies (29, 30). Thus, it logically follows to use S protein as an antigen for vaccine development (30). Recently, it has been shown that the S protein of SARS-CoV is a major inducer of protective immunity among structural proteins (31). Yang, et al. (32) reported that a DNA vaccine candidate encoding the S protein induced SARS-CoV neutralization (neutralizing antibody titers ranged from 1:25 to 1:150) and protective immunity in mice, and it was proven that the protection was mediated by neutralizing antibodies but not by a T-cell-dependent mechanism. Bisht, et al. (33) demonstrated that the S protein of SARS-CoV expressed by attenuated vaccinia virus (MVA) elicited S-specific antibodies with SARS-CoV-neutralizing antibody titer of 1:284, and protectively-immunized mice against SARS-CoV infection as shown by reduced titers of SARS-CoV in the respiratory tracts of mice after the challenge infection. Bukreyev, et al. (34) reported that mucosal immunization of African green monkeys with an attenuated parainfluenza virus (BHPIV3) expressing the SARS-CoV S protein induced neutralizing antibodies with neutralization titers ranging from 1:8 to 1:16 and protected animals against the challenge infection. These data indicate that the S protein of SARS-CoV is a protective antigen capable of inducing neutralizing antibodies, although its antigenic determinants remain to be defined.

We have recently demonstrated that the receptor-binding domain (RBD) of SARS-CoV S protein is a major target of neutralizing antibodies induced in patients infected with SARS-CoV and in animals immunized with inactivated viruses or S proteins (35,36). Therefore, we used the recombinant RBD of the SARS-CoV S protein as an immunogen to induce neutralizing monoclonal antibodies (mAbs).

### BRIEF SUMMARY OF THE INVENTION

According to the present invention, there is provided an isolated neutralising monoclonal antibody capable of binding to the receptor-binding domain of the spike protein of the severe acute respiratory syndrome-associated coronavirus (SARS-CoV) wherein the antibody is produced by Hybridoma 30F9 with ATCC Accession No. PTA-6523.

Further according to the invention, there is provided a pharmaceutical composition comprising an effective amount of the antibody according to the invention and a pharmaceutically-acceptable carrier.

Further according to the invention, there is provided Hybridoma 30F9 with ATCC Accession No. PTA-6523.

Further according to the invention, there is provided an antibody according to the invention for use in the treatment of SARS-CoV infection.

Further according to the invention, there is provided the use of an antibody according to the invention in the preparation of a medicament for the treatment of SARS-CoV infection.

Further according to the invention, there is provided a method for detecting severe acute respiratory syndrome-associated coronavirus (SARS-CoV) or SARS-CoV-infected cells *in vitro,* comprising contacting an antibody according to the invention under conditions permitting the formation of complexes between the antibody and the receptor-binding domain of the spike protein of the SARS-CoV, and detecting the complexes formed.

Further according to the invention, there is provided a method for screening compounds capable of inhibiting infection by severe respiratory syndrome-associated coronavirus (SARS-CoV) by blocking the binding of the virus to receptors on host cells, comprising the steps of:
(a) establishing a system for an antibody according to the invention to bind to the receptor-binding domain of the spike protein of the SARS-CoV; and
(b) contacting a compound to be screened with the system of (a), a decrease in binding of the antibody to the receptor-binding domain of the spike protein of the SARS-CoV indicating that the compound is capable of interfering with the binding and inhibiting infection of the receptor-binding domain of the spike protein of SARS-CoV.

Further according to the invention, there is provided a diagnostic kit or a screening kit comprising a compartment containing an antibody according to the invention.

The present invention demonstrates that the receptor-binding domain (RBD) contains multiple, conformation-dependant, neutralization epitopes which induce a panel of potent neutralizing monoclonal antibodies (mAbs), which can be used for the treatment, diagnosis, and prevention of SARS.

### -BRIEF DESCRIPTION OF THE SEVERAL VIEWS OF THE DRAWINGS

Figure. 1. (not according to the invention) Epitope mapping of mAbs 4D5 and 17H9 by overlapping peptides that cover the RBD of S protein. Each of the peptides was coated at 5 µg/ml and mAbs were tested at 10 µg/ml.
Figure. 2. Inhibition of RBD-Fc binding to ACE2 by mAbs. Upper panel shows inhibition of RBD-Fc binding to cell-associated ACE2 expressed on 293T/ACE2 cells measured by flow cytometry; lower panel shows inhibition of RBD-Fc binding to soluble ACE2 measure by ELISA. RBD-Fc was used at 1 µg/ml and mAbs were used at 50 µg/ml. % inhibition was calculated for each mAb.
Figure. 3. Neutralization of SARS pseudovirus by mAbs. Inhibition of SARS pseudovirus infection in 293T/ACE2 cells by representative mAbs from each group was shown. Each of the mAbs was tested at a series of 2-fold dilutions and % neutralization was calculated.

### DETAILED DESCRIPTION OF THE INVENTION

The present invention provides an isolated monoclonal antibody capable of binding to receptor-binding domain (RBD) of the spike (S) protein of the severe acute respiratory syndrome-associated coronavirus (SARS-CoV) so as to competitively inhibit the binding of the SARS-CoV to host cells.

The present invention (not according to the invention) also provides a substance comprising the complementary-determining regions of the monoclonal antibody described above, capable of binding to the same epitope as the monoclonal antibody described above. This substance includes, but is not limited to, a polypeptide, small molecule, antibody, or a fragment of an antibody. In a preferred embodiment, the antibody is neutralizing. In another embodiment, the antibody is a single-chain antibody or antibody-fusion construct; a humanized antibody; or a chimeric antibody as described above. It is the intention of this application to cover different chimeric constructs created using the invented antibodies. The present invention also covers all the humanized constructs of the antibodies. The art of generating chimeric or humanized antibodies is well-known. See eg. (37, 38) for chimeric antibodies and (39-41) for humanized antibodies. An ordinarily-skilled artisan may modify the sequence of the above described substance in light of the present disclosure. Said modification may include addition, deletion, or mutation of certain amino acid sequences in the fragment. The general method to produce an antibody is within the knowledge of one of ordinary skill in the art. See e.g., Using Antibodies: A Laboratory Manual: Portable Protocol No. 1 by Ed. Harlow (1998).

In one embodiment, the isolated antibody described above is directly or indirectly coupled to one of more cytotoxic agent. Said cytotoxic agent includes, but is not limited to, radionucleotides or other toxins. The present invention (not according to the invention) additionally provides a nucleic acid molecule encoding the above antibody. Once the antibodies are isolated, the gene which encodes said antibody may be isolated and the nucleic acid sequence will be determined. Accordingly, the present invention (not according to the invention) further provides a nucleic acid molecule capable of specifically hybridizing the molecule described above. The nucleic acid molecule includes, but is not limited to, synthetic DNA or RNA, genomic DNA, cDNA, and RNA.

The present invention (not according to the invention) also provides a vector comprising the above nucleic acid molecules or a portion thereof. This portion may be a functional portion which carries out a certain function. A fragment or a partial sequence may be able to encode a functional domain of the protein which is functional. In one embodiment, this vector is an expression vector, whereby the protein encoded by nucleic acid molecule may be expressed. The present invention further provides a cell comprising the above-described nucleic acid molecule. Said cells may be used for expression. Vectors are well-known in this field. See e.g., Graupner, U.S. Patent No. 6,337,208, "Cloning Vector," issued January 8, 2002. See also, Schumacher et. al., U.S. Patent No. 6,190,906, "Expression Vector for the Regulatable Expression of foreign Genes in Prokaryotes," issued February 20, 2001. In one embodiment, the vectors are plasmids. The present invention provides a method for producing the antibody capable of binding to receptor-binding domain (RBD) of the spike (S) protein of the SARS-CoV so as to competitively inhibit the binding of the SARS-CoV to host cells, comprising operatively-linking the nucleic acid molecule described above to appropriate regulatory element so as to express said antibody; placing the linked nucleic molecule in appropriate conditions permitting the expression of said antibody; and recovery of said expressed antibody, thereby producing said antibody. The present invention also provides an antibody produced by the above method.

Hybridoma cell lines 32H5 (Conf I), 31H12 (Conf II), 18D9 (Conf III), 30F9 (Conf IV), 33G4 (Conf V), and 19B2 (Conf VI) were deposited on January 13, 2005 with American Type Culture Collection (ATCC), 10801 University Blvd., Manassas, VA 20110, U.S.A., under the provisions of the Budapest Treaty for the International Recognition of the Deposit of Microorganism for the Purposes of Patent Procedure. Cell lines 32H5 (Conf I), 31H12 (Conf II), 18D9 (Conf III), 30F9 (Conf IV) (according to the invention), 33G4 (Conf V), and 19B2 (Conf VI) were accorded ATCC Accession Numbers PTA-6525, PTA-6524, PTA-6521, PTA-6523, PTA-6526, and PTA-6522, respectively.

The present invention (not according to the invention) also provides epitopes recognized by the above-described monoclonal antibodies. Said epitopes, sequential or conformational, are important for diagnostic or therapeutic uses.

The present invention provides a. composition comprising an effective amount of the above-described monoclonal antibody according to the invention and a suitable carrier. The effective amount may be determined by routine experimentation. The present invention additionally provides a pharmaceutical composition comprising an effective amount of the above-described monoclonal antibody and a pharmaceutically-acceptable carrier. As used herein, a pharmaceutieally-acceptable carrier means any of the standard pharmaceutical carriers. Examples of suitable carriers are well-known in the art and may include, but are not limited to, any of the standard pharmaceutical carriers such as a phosphate buffered saline solutions, phosphate buffered saline containing Polysorb 80, water, emulsions such as oil/water emulsion, and various types of wetting agents. Other carriers may also include sterile solutions, tablets, coated tablets, and capsules. Typically such carriers contain excipients such as starch, milk, sugar, certain types of clay, gelatin, stearic acid or salts thereof, magnesium or calcium stearate, talc, vegetable fats or oils, gums, glycols, or other known excipients. Such carriers may also include flavor and color additives or other ingredients. Compositions comprising such carriers are formulated by wen-known conventional methods.

The present invention further provides a method for detecting SARS-CoV (or the SARS-CoV-infected cells), comprising contacting the antibody according to the invention under conditions permitting the formation of complexes between the antibody and the RBD of the S protein of the SARS-CoV; and detecting the complexes formed.

Finally, the present invention provides a method for screening compounds capable of inhibiting infection of SARS-CoV by blocking the binding of said virus to receptors on host cells, comprising the steps of (a) establishing a system for the antibody of the invention to bind to the receptor-binding domain (RBD) of the spike (S) protein of the SARS-CoV; and (b) contacting the compounds with the system of (a), whereby a decrease in binding of the above antibody to the RBD of the S protein of the SARS-CoV indicates that the compounds are capable of interfering with said binding, thereby inhibiting infection of the RBD of the S protein of the SARS-CoV.

The present invention provides a kit comprising a compartment containing an antibody according to the invention capable of recognizing the SARS virus and/or a substance which can competitively inhibit the binding of said antibody.

The present invention demonstrates that the RBD contains multiple conformation-dependant neutralization epitopes which induce a panel of potent neutralizing monoclonal antibodies (mAbs), which can be used for the treatment, diagnosis and prevention of SARS.

The invention will be better understood by reference to the Experimental Details which follow. **Experimental Details**

Twenty-seven hybridoma clones were generated by fusing SP2/0 myeloma cells with the splenocytes from Balb/c mice immunized with a fusion protein containing a receptor-binding domain (RBD) in the spike (S) protein of the SARS-CoV linked to human IgG1 Fc fragment (designated RBD-Fc). Among the 27 monoclonal antibodies (mAbs) produced from these hybridoma clones, except 2 mAbs bound to the adjacent linear epitopes, all other mAbs recognized conformation-dependant epitopes. Based on the results obtained from binding competition experiments, these 25 conformation-specific mAbs could be divided into six groups, designated as Conf I-VI. The Conf IV and Conf V mAbs significantly blocked RBD-Fc binding to ACE2, the receptor for SARS-CoV, suggesting that their epitopes overlap with the receptor-binding sites in the S protein. Most of the mAbs (23/25) that recognize the conformational epitopes possessed potent neutralizing activities against SARS pseudovirus with 50% neutralizing dose (ND₅₀) raging from 0.005 to 6.569 µg/ml.

These SARS-CoV neutralizing mAbs can be used: 1) as immunotherapeutics for early treatment of SARS-CoV infection; 2) as biological reagents for diagnosis of SARS-CoV infection; and 3) as probes for studying the immunogenicity, antigenicity, structure and function of the SARS-CoV S protein. Furthermore, these murine mAbs can be humanized for therapy and prevention of SARS-CoV infection.

### Materials and methods

*Immunization of mice and generation of mAbs.* Five Balb/c mice (4 wks old) were immunized subcutaneously with 20 µg of Protein A Sepharose-purified RBD-Fc prepared as previously described (35) in the presence of MLP+TDM Adjuvant System (Sigma, Saint Louis, MI) and boosted with 10 µg of the same antigen plus the MLP+TDM adjuvant at 3-wk intervals. Mouse antisera were collected for detecting anti-RBD antibodies and SARS-CoV-neutralizing antibodies.

Hybridomas for producing anti-RBD mAbs were generated using standard protocol. Briefly the splenocytes from the immunized mice were harvested and fused with SP2/0 myeloma cells. Cell culture supernatants from the wells containing hybridoma colonies were screened by enzyme-linked immunosorbent assay (ELISA) using S1-C9 prepared as previously described (35) as a coating antigen. Cells from positive wells were expanded and retested. Cultures that remained positive were subcloned to generate stable hybridoma cell lines. All mAbs were purified from culture supernatants by Protein A Sepharose 4 Fast Flow (Amersham Biosciences).

*ELISA. and binding competition.* Reactivity of mouse sera or mAbs with various antigens was determined by ELISA. Briefly, 1 µg/ml recombinant proteins (RBD-Fc or S1-C9) or purified human IgG (Zymed, South San Francisco, CA) were used, respectively, to coat 96-well microtiter plates (Coming Costar, Acton, MA) in 0.1 M carbonate buffer (pH 9.6) at 4 °C overnight. After blocking with 2% non-fat milk, serially diluted mouse sera or mAbs were added and incubated at 37°C for 1 h, followed by four washes with PBS containing 0.1% Tween 20. Bound antibodies were detected with HRP-conjugated goat anti-mouse IgG (Zymed) at 37 °C for 1h, followed by washes. The reaction was visualized by addition of the substrate 3,3',5,5'-tetramethylbenzidine (TMB) and absorbance at 450 nm was measured by an ELISA plate reader (Tecan US, Research Triangle Park, NC).

To determine the effect of disulfide bond reduction on the binding of RBD-specific mAbs, ELISA plate was coated with recombinant RBD-Fc or S1-C9 at a concentration of 1 µg/ml and then treated for 1 h at 37°C with dithiothreitol (DTT) at a concentration of 10 mM, followed by washes. Then the wells were treated with 50 mM iodoacetamide for 1 h at 37°C. After washes, a standard ELISA was performed as described above.

A competitive ELISA was performed to determine the inhibitory activity of the RBD-specific mAbs on binding of the biotinylated mAbs to RBD-Fc. Briefly, the wells of ELISA plates were coated with RBD-Fc at 1 µg/ml as described above. A mixture containing 50 µg/ml of an unlabeled mAb and 1 µg/ml of a biotinylated mAb was added, followed by incubation at 37 °C for 1 h. Binding of the biotinylated mAbs was detected after addition of HRP-conjugated streptavidin (Zymed) and TMB sequentially. Biotinylation of mAbs was performed using the EZ-link NHS-PEO Solid Phase Biotinylation Kit (Pierce, Rockford, IL) according to the manufacturer's protocol.

*Neutralization of SARS pseudovirus infection.* The conventional neutralization assay using live SARS-CoV is cumbersome and has to be performed in BSL-3 facilities. We therefore adapted a SARS-CoV pseudovirus system (27, 32, 42, 43) in our laboratory. This assay is sensitive and quantitative and can be carried out in BSL-2 facilities. SARS pseudovirus bearing SARS-CoV S protein and a defective HIV-1 genome that expresses luciferase as reporter was prepared as previously described (27, 42, 43). In brief, 293T cells were co-transfected with a plasmid encoding codon-optimized SARS-CoV S protein and a plasmid encoding Env-defective, luciferase-expressing HIV-1 genome (pNL4-3.1uc.RE)using Fugene 6 reagents (Boehringer Mannheim). Supernatants containing SARS pseudovirus were harvested 48 hours post-transfection and used for single-cycle infection of ACE2-transfected 293T (293T/ACE2) cells. Briefly, 293T/ACE2 cells were plated at 10⁴ cells/well in 96-well tissue-culture plates and grown overnight. The supernatants containing pseudovirus were preincubated with 2-fold serially diluted mouse sera or mAbs at 37°C for 1 h before addition to cells. The culture was re-fed with fresh medium 24 h later and incubated for an additional 48 h. Cells were washed with PBS and lysed using lysis reagent included in a luciferase kit (Promega, Madison, WI). Aliquots of cell lysates were transferred to 96-well Costar flat-bottom luminometer plates (Corning Costar, Coming, NY), followed by addition of luciferase substrate (Promega). Relative light units (RLU) were determined immediately in the Ultra 384 luminometer (Tecan US).

*Binding inhibition of RBD-Fc with receptor by mAbs.* Inhibition of mAbs on RBD-Fc binding to ACE2-expressing cells was measured by flow cytometry. Briefly, 10⁶ 293T/ACE2 cells were detached, collected, and washed with Hank's balanced salt solution (HBSS) (Sigma, St Louis, MO). RBD-Fc was added to the cells to a final concentration of 1 µg/ml in the presence or absence of 50 µg/ml mAbs, followed by incubation at room temperature for 30 min. Cells were washed with HBSS and incubated with anti-human IgG-FITC conjugate (Zymed) at 1:50 dilution at room temperature for an additional 30 min. After washing, cells were fixed with 1% formaldehyde in PBS and analyzed in a Becton FACSCalibur flow cytometer (Mountain View, CA) using CellQuest software.

Inhibition of RBD-Fc binding to soluble ACE2 by mAbs was measured by ELISA. Briefly, recombinant soluble ACE2 (R&D systems, Inc., Minneapolis, MN) at 2 µg/ml was coated onto 96-well ELISA plates (Coming Costar) in 0.1 M carbonate buffer (pH 9.6) at 4 °C overnight. After blocking with 2% non-fat milk, 1 µg/ml RBD-Fc was added to the wells in the presence or absence of 50 µg/ml mouse mAbs and incubated at 37°C for 1 hour. After washing, the HRP-conjugated goat anti-human IgG (Zymed) was added and incubated an additional 1 h. After washing, the substrate TMB was used for detection.

### Results

*Isolation and initial characterization of mAbs specific for RBD.* RBD-Fc fusion protein was transiently expressed in 293T cells and purified to homogenicity by Protein A. Five mice (A to E) were immunized four times with RBD-Fc in the presence of Ribi adjuvant. All animals developed appreciable antibody responses against RBD-Fc after the first boost, and their antibody titers increased with subsequent immunizations. The antisera collected 4 days after the third boost showed highly potent neutralizing activity against SARS-CoV and SARS pseudovirus bearing SARS-CoV S protein.

A panel of 27 RBD-specific mAbs was generated by fusing splenocytes from the RBD-Fc-immunized mice with Sp2/0 myeloma cells and then screening hybridomas with S1-C9 as an antigen. Epitope specificities of these mAbs were initially determined by ELISAs using RBD-Fc, DTT-reduced RBD-Fc, S1-C9, DTT-reduced S1-C9, and a purified human IgG as coating antigens (Table I). Majority of the mAbs (25/27) were reactive with native RBD-Fc and S1-C9, but not DTT-reduced RBD-Fc and S1-C9. This indicated that they were directed against disulfide bond-dependent conformational epitopes expressed on the RBD of S protein. Other two mAbs (4D5 and 17H9) recognized both native and reduced RBD-Fc and S I -C9, indicating that they were directed against linear epitopes presented on the RBD. None of the mAbs screened by S1-C9 reacted with human IgG, whereas control antiserum from a mouse immunized with RBD-Fc was reactive with human IgG (Table I).

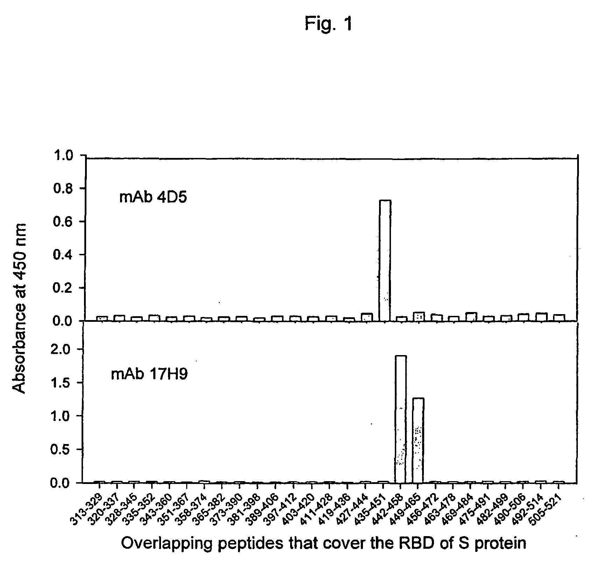

Since the mAbs 4D5 and 17H9 could react with the reduced RBD-Fc and S1-C9, their epitopes might be mapped with synthetic peptides. A set of 27 overlapping peptides that cover the RBD of S protein was used to localize 4D5 and 17H9 epitopes by ELISA. As shown in Fig. 1, 4D5 reacted with the peptide 435-451 (NYNYKYRYLRHGKLRPF), and 17H9 reacted with two overlapped peptides 442-458 (YLRHGKLRPFERDISNV) and 449-465 (RPFERDISNVPFSPDGK). While the epitope of 17H9 was clearly mapped to the overlapped sequence (RPFERDISNV) of the peptides 442-458 and 449-465, the epitope for 4D5 requires most sequence of the peptide 435-451 which overlaps partial sequences of the peptides 442-458 and 449-465. Therefore, these two mAbs recognize neighboring linear epitopes that reside within the RBD. None of the conformation-dependant mAbs reacted with any of the tested peptides (data not shown).

*Epitope specificity of the RBD-specific mAbs determined by binding competition assays.* In order to characterize the conformation-dependant epitopes, the RBD-specific mAbs were grouped by binding competition assays (Table II). One of the mAbs (10E7) was first biotinylated and the inhibitory activity of the 27 mAbs on 10E7 binding to RBD-Fc was measured. The mAbs 4D5 and 17H9 recognizing linear epitopes mapped by peptides above were included in the competition assays as a control. About half of the conformation-dependant mAbs (13/25) competed with biotinylated 10E7, while other mAbs did not block 10E7 binding to R.BD-Fc. Another four of the non-competing mAbs (11 E12, 33G4, 45B5, and 17H9) were subsequently biotinylated and tested similarly with the binding competition assay. Five of the 13 mAbs that compete with the biotinylated 10E7 also blocked the biotinylated 45B5 binding to RBD-Fc and were designated as a separate group. Thus, the 25 conformation-specific mAbs were divided into six distinct competition groups (designated as Conf I-VI). Two linear epitope-specific mAbs (4D5 and 17H9) did not compete with any of conformation-specific mAbs. These results suggest that the RBD of S protein contains multiple antigenic structures that induce specific antibody responses in the mice. However, the immunodominant epitopes in the RBD are conformation-dependant.

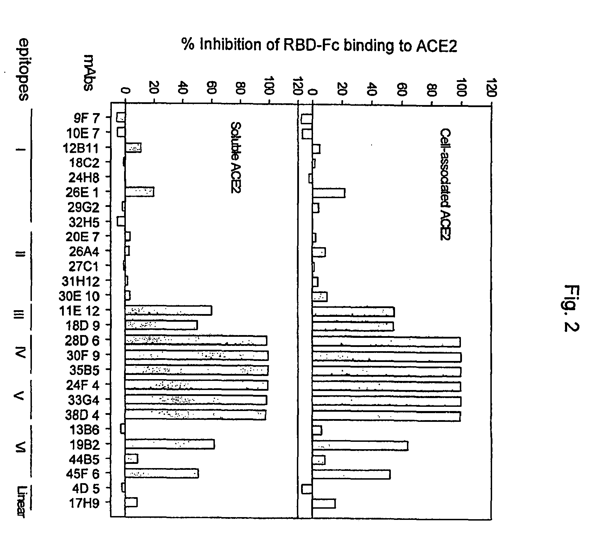

*Characterization of the mAbs that block receptor binding.* RBD-Fc could efficiently bind to ACE2 expressed on 293T/ACE2 cells and to soluble ACE2 as measured by flow-cytometry and ELISA, respectively (data not shown). We tested whether the RBD-specific mAbs inhibit binding of RBD-Fc to cell-associated or soluble ACE2. As shown in Fig. 2, all of the mAbs from Conf IV (28D6, 30F9, and 35B5) and Conf V (24F4, 33G4, and 38D4) completely blocked RBD-Fc binding to both cell-associated and soluble ACE2 in a highly consistent manner. All the two Conf III mAbs (11E12 and 18D9) and two of the four Conf VI mAbs (19B2 and 45F6) partially inhibited RBD-Fc binding to ACE2 expressed on 293T/AEC2 cells and soluble ACE2. All of other mAbs, including two mAbs against linear sequences, had no significant inhibitory effects on receptor binding. These results indicate that the Conf IV and Conf V mAbs recognize epitopes that may overlap with the conformational receptor-binding sites in the S protein, although these mAbs did not compete against each other in the binding competition assays. Conf III mAbs and two Conf VI mAbs (19B2 and 45F6) may also bind to the conformational epitopes being involved in the receptor-binding. All the Conf I and Conf II mAbs did not block the receptor binding, suggesting that they recognize the conformational epitopes that do not overlap the receptor-binding sites in RBD. These results highlight the epitopic heterogenecity of the RBD-specific mAbs and further indicate that the RBD of S protein contains multiple antigenic conformations.

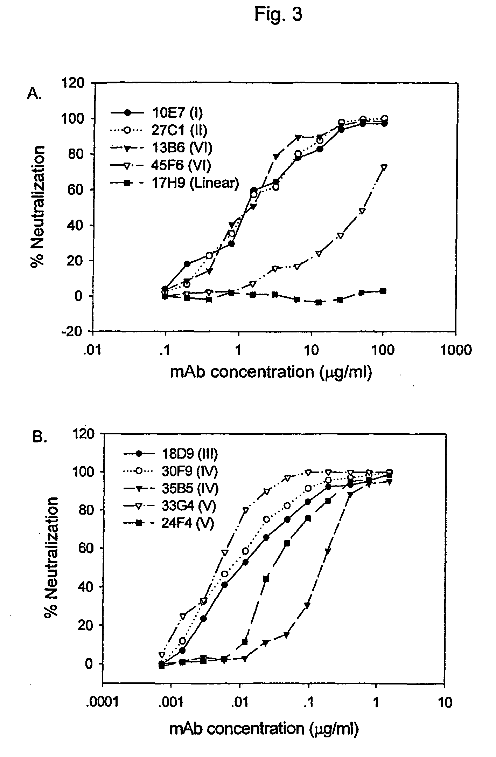

*RBD-specific mAbs have potent neutralizing activity.* Each of the RBD-specific mAbs was tested for neutralizing activity against SARS pseudovirus. Strikingly, the majority of the conformation-dependant mAbs (23/25) had potent neutralizing activity with 50% neutralization dose (ND₅₀) ranging from 0.005 to 6.569 µg/ml (Table III), whereas two mAbs that direct against linear epitopes (4D5 and 17H9) and one mAb from Conf VI (44B5) at a concentration as high as 100 µg/ml did not neutralize the SARS pseudovirus infection. The mAbs 33G4 from Conf V and 30F9 from Conf IV that blocked the receptor binding had highest neutralizing activities against the pseudovirus. Interestingly, even 45F6 from Conf VI, with its relatively lower pseudovirus neutralization activity, partially blocked the binding of RBD-Fc with ACE2. The dose-dependent neutralizing activity of several representative mAbs from each of groups was presented in Fig. 3. These results suggest that the RBD of S protein predominantly induce neutralizing antibodies that direct against conformational epitopes.

### Experimental Discussion

Recent studies have shown that the S protein of SARS-CoV is one of the major antigens eliciting immune responses during infection (44-46). These suggest that the S protein may serve as an immunogen for inducing neutralizing mAbs. In the present study, we used a recombinant fusion protein RBD-Fc as an immunogen to immunize mice and generated hybridoma clones to produce 27 mAbs. A majority of these mAbs (25/27) recognized conformational epitopes and among them, 23 mAbs had potent neutralizing activity. Only two mAbs were mapped to adjacent linear epitopes by overlapping peptides and they could not neutralize infection by SARS pseudovirus. Interestingly, the conformation-dependant mAbs could be divided into six different groups (*i.e.,* Conf I-VI) based on a binding competition experiment, suggesting that there are several distinct conformational epitopes on the RBD that can elicit neutralizing antibodies.

It is expected that all the neutralizing mAbs directed against the RBD can block the interaction between RBD and ACE2, the functional receptor for SARS-CoV. However, we found that only the mAbs recognizing the Conf IV and V could efficiently block RBD binding to ACE2. Some mAbs reacting with the Conf III and VI partially inhibited interaction between the RBD and ACE2. This suggests that their epitopes may overlap the receptor-binding sites on the RBD or binding of these mAbs to RBD may cause conformational change of the receptor binding sites, resulting in inhibition of RBD binding to ACE2. The mAbs that recognize the Conf I and II did not significantly affect the RBD binding with ACE2, but also possessed potent neutralizing activities, suggesting that these mAbs inhibit SARS-CoV infection without interfering in RBD-ACE2 interaction. The mechanism of action of these mAbs needs to be further investigated. These data indicate that the RBD induces neutralizing antibodies specific not only for the receptor-binding sites, but also for other unique structural conformations, highlighting its antigenic heterogenicity, and suggest that the RBD of SARS-CoV S protein contains multiple conformational epitopes responsible for induction of potent neutralizing antibody responses.

The conformational sensitivity of the SARS-CoV neutralizing mAbs described here is consistent with properties of neutralizing mAbs raised against other enveloped viruses, which generally require more native conformation for binding (47, 48). Although the RBD of SARS-CoV S protein is a 193-amino-acid small fragment, it contains seven cysteines and five of which are essential for ACE2 association. The disulfide-bonds between these cysteines may form complex tertiary structures to constitute the multiple antigenic conformations. However, a neutralizing human mAb selected from a nonimmune human antibody library could react with the DTT-reduced S protein and block receptor association (49). Therefore, further characterization is needed to define the neutralization determinants on the RBD of SARS-CoV S protein, and this may provide critical information for developing anti-SARS therapeutics and vaccines.

It was reported that passive transfer of mouse immune sera reduced pulmonary viral replication in the mice challenged with SARS-CoV (33, 50), and prophylactic administration of neutralizing mAbs conferred *in vivo* protection in the mice or in the ferrets (51, 52), suggesting that passive immunization with anti-SARS antibodies is a viable strategy to control SARS. Thus, mAbs with high levels of SARS-CoV neutralizing activity may be used for early treatment of SARS-CoV infection. However, application of murine MAbs in human will be limited due to human-anti-mouse antibody (HAMA) responses (53-55). If only a few doses of murine mAbs are used in a short period of time (one to two weeks) at the early stage of SARS-CoV infection may not cause serious HAMA, but this urgent treatment may save lives of SARS patients. We have used similar strategies for early treatment of Hantaan virus (HTNV) infection using murine anti-HTNV mAbs(56). Furthermore, the murine neutralizing mAbs can be humanized as therapeutics or immunoprophylaxis for providing immediate protection against SARS-CoV infection to those at-risk populations.

The significance of the present study is three-fold. First, a number of highly potent RBD-specific neutralizing mAbs have been generated, which may be developed as immunotherapeutics for urgent SARS treatment. Second, these mAbs can be developed as diagnostic agents for detecting SARS-CoV infection. Third, these mAbs can be used as probes for studying the immunogenicity, antigenicity, structure, and function of the SARS-CoV S protein. These mAbs can be further humanized for treatment and prevention of SARS.

**Table I. Reactivities of RBD-specific mAbs against various antigens^{a}**

| **mAbs** | **Isotype** | **Antigen** | | | | |
|---|---|---|---|---|---|---|
| | | RBD-Fc | Reduced RBD-Fc | S1-C9 | **Reduced S1-C9** | **Human IgG** |
| 4D5 | IgG1/κ | **0.88** | **1.36** | **0.65** | **0.94** | 0.02 |
| 9F7 | IgG1/κ | **1.60** | 0.00 | **1.45** | 0.08 | 0.04 |
| 10E7 | IgG1/κ | **1.77** | 0.02 | **1.72** | 0.16 | 0.05 |
| 11E12 | IgG2a/κ | **1.50** | 0.01 | **0.72** | 0.09 | 0.02 |
| 12B11 | IgG1/κ | **1.37** | 0.04 | **0.78** | 0.00 | -0.01 |
| 13B6 | IgG1/κ | **1.58** | -0.01 | **0.93** | 0.00 | 0.02 |
| 17H9 | IgG1/κ | **1.72** | **1.71** | **1.21** | **1.15** | 0.07 |
| 18C2 | IgG1/κ | **1.28** | -0.01 | **0.80** | 0.01 | -0.20 |
| 18D9 | IgG1/κ | **1.47** | -0.01 | **0.90** | 0.01 | 0.03 |
| 19B2 | IgG1/κ | **1.63** | 0.00 | **1.55** | 0.12 | 0.01 |
| 20E7 | IgG2a/κ | **1.50** | 0.00 | **0.98** | 0.01 | 0.02 |
| 24F4 | IgG1/κ | **1.69** | -0.01 | **1.08** | 0.08 | 0.04 |
| 24H8 | IgG1/κ | **1.54** | -0.01 | **0.94** | 0.12 | 0.01 |
| 26A4 | IgG1/κ | **1.60** | 0.00 | **0.89** | 0.09 | 0.01 |
| 26E1 | IgG1/κ | **1.91** | 0.07 | **1.85** | 0.06 | 0.01 |
| 27C1 | IgG1/κ | **1.46** | 0.00 | **1.57** | 0.07 | 0.01 |
| 28D6 | IgG1/κ | **2.06** | 0.01 | **1.60** | 0.16 | 0.00 |
| 29G2 | IgG2a/κ | **1.69** | 0.00 | **0.96** | 0.17 | 0.04 |
| 30F9 | IgG1/κ | **1.66** | 0.04 | **1.21** | 0.12 | 0.01 |
| 31H12 | IgG1/κ | **1.72** | 0.08 | **1.91** | 0.22 | 0.03 |
| 32H5 | IgG1/κ | **1.54** | 0.06 | **1.55** | 0.51 | 0.00 |
| 33G4 | IgG2a/κ | **1.79** | 0.02 | **1.76** | 0.20 | -0.01 |
| 34E10 | IgG1/κ | **1.62** | 0.10 | **1.82** | 0.18 | 0.04 |
| 35B5 | IgG1/κ | **1.74** | 0.06 | **1.72** | 0.25 | 0.02 |
| 38D4 | IgG1/κ | **1.63** | -0.01 | **1.20** | 0.07 | 0.00 |
| 44B5 | IgG1/κ | **1.57** | 0.09 | **1.64** | 0.16 | 0.00 |
| 45F6 | IgG1/κ | **1.61** | 0.11 | **1.43** | 0.15 | -0.01 |
| Antiserum | | **2.22** | **1.78** | **2.32** | **1.68** | **2.07** |
| Naïve serum | | 0.01 | 0.02 | 0.02 | 0.01 | 0.04 |

| | | | | | | |
|---|---|---|---|---|---|---|
| ^{a} Antigens were used at 1 µg/ml; mAbs were tested at 10µg/ml and sera were tested at 1:100 dilution. Positive reactivities are highlighted in boldface. | | | | | | |

**Table II. % Inhibition of RBD-specific mAbs on binding of biotinylated mAbs to RBD-Fc^{a}**

| Group | Competing mAb | Biotinylated mAb | | | | |
|---|---|---|---|---|---|---|
| | | 10 E7 | 11 E12 | 33G4 | 45B5 | 17H9 |
| Conf I | 9F7 | **84.5** | 11.7 | -13.3 | 22.3 | 16.4 |
| | 10E 7 | **91.0** | 5.6 | -12.9 | 21.0 | 9.9 |
| | 12B11 | **85.8** | 19.3 | -0.2 | 19.8 | 21.0 |
| | 18C2 | **84.9** | 19.3 | 4.9 | 18.1 | 19.4 |
| | 24H8 | **93.7** | 24.0 | 7.0 | 25.6 | 22.1 |
| | 26E1 | **95.1** | 10.5 | 37.4 | 30.4 | 25.0 |
| | 29G2 | **96.6** | 20.4 | 1.6 | 11.4 | 23.5 |
| | 32H5 | **98.9** | 18.5 | 4.4 | 9.1 | 20.3 |
| | | | | | | |
| Conf II | 20E7 | **97.2** | 38.5 | 5.9 | **73.0** | 24.6 |
| | 26A4 | **96.3** | 33.1 | -0.5 | **60.0** | 19.0 |
| | 27C1 | **97.2** | 36.7 | 14.6 | **73.7** | 20.9 |
| | 31H12 | **97.5** | 18.7 | 7.1 | **58.4** | 19.7 |
| | 30E10 | **98.3** | 19.3 | 12.9 | **68.9** | 24.6 |
| | | | | | | |
| Conf III | 11E12 | 12.6 | **92.0** | 0.3 | -3.7 | 20.2 |
| | 18D9 | -16.2 | **98.3** | 8.3 | 23.6 | 17.1 |
| | | | | | | |
| Conf IV | 28D6 | 39.7 | **99.6** | 13.8 | **67.4** | 26.6 |
| | 30F9 | 28.7 | **100.0** | 8.7 | **64.0** | 32.4 |
| | 35B5 | 34.9 | **99.9** | 10.0 | **64.7** | 33.6 |
| | | | | | | |
| Conf V | 24F4 | 11.5 | -1.0 | **95.5** | 2.5 | 24.9 |
| | 33G4 | 9.5 | -3.7 | **99.5** | 26.4 | 29.1 |
| | 38D4 | 8.1 | -14.4 | **82.0** | -5.1 | 15.8 |
| | | | | | | |
| Conf VI | 13B6 | 23.3 | 10.7 | -4.9 | **72.5** | 12.6 |
| | 19B2 | 2.9 | -26.4 | 18.0 | **50.0** | 16.1 |
| | 44B5 | 25.3 | -20.6 | 10.0 | **95.6** | 19.4 |
| | 45F6 | 25.7 | -10.4 | 10.8 | **94.8** | 23.5 |
| | | | | | | |
| Linear | 4D5 | 13.0 | 10.6 | -11.1 | 1.0 | -10.5 |
| | 17H9 | 17.8 | 33.3 | -5.8 | 25.0 | **97.8** |

| | | | | | | |
|---|---|---|---|---|---|---|
| ^{a}Competing mAbs were tested at 100 µg/ml for the ability to block binding of the biotinylated mAbs to the RBD-Fc in ELISA. Greater than 40% inhibition was considered positive competition (values in bold). Negative numbers indicate increased binding of the biotinylated reagent | | | | | | |

**Table III. Neutralization activity of RBD-specific mAbs against SARS pseudovirus**

| Group | mAb | Inhibition of ACE2 binding^{a} | ND₅₀ (µg/ml) |
|---|---|---|---|
| Conf I | 9F7 | - | 6.569 |
| | 10E 7 | - | 1.673 |
| | 12B11 | - | 4.918 |
| | 18C2 | - | 5.031 |
| | 24H8 | - | 3.955 |
| | 26E1 | - | 0.354 |
| | 29G2 | - | 3.02 |
| | 32H5 | - | 0.275 |
| | | | |
| Conf II | 20E7 | - | 5.959 |
| | 26A4 | - | 2.815 |
| | 27C1 | - | 1.607 |
| | 31H12 | - | 0.139 |
| | 30E10 | - | 0.399 |
| | | | |
| Conf III | 11E12 | + | 1.39 |
| | 18D9 | + | 0.02 |
| | | | |
| Conf IV | 28D6 | ++ | 0.298 |
| | 30F9 | ++ | 0.009 |
| | 35B5 | ++ | 0.131 |
| | | | |
| Conf V | 24F4 | ++ | 0.052 |
| | 33G4 | ++ | 0.005 |
| | 38D4 | ++ | 0.332 |
| | | | |
| Conf VI | 13B6 | - | 1.436 |
| | 19B2 | + | 0.936 |
| | 44B5 | - | >100 |
| | 45F6 | + | 43.894 |
| | | | |
| Linear | 4D5 | - | >100 |
| | 17H9 | - | >100 |

| | | | |
|---|---|---|---|
| ^{a} "-," "+,"and "++" indicate no, partial, and complete inhibition, respectively. | | | |

### References

1. Drosten, C., S. Gunther, W. Preiser, W.S. Van Der, H.R. Brodt, S. Becker, H. Rabenau, M. Panning, L. Kolesnikova, RA. Fouchier, A. Berger, A.M. Burguiere, J. Cinatl, M. Eickmann, N. Escriou, K. Grywna, S. Kramme, J.C. Manuguerra, S. Muller, V. Rickerts, M. Sturmer, S. Vieth, H.D. Klenk, A.D. Osterhaus, H. Schmitz, and H. W. Doerr. 2003. Identification of a novel coronavirus in patients with severe acute respiratory syndrome. N. Engl. J. Med 348:1967-1976.
2. Ksiazek, T.G., D. Erdman, C.S. Goldsmith, S.R Zaki, T. Peret, S. Emery, S. Tong, C. Urbani, J.A. Comer, W. Lim, P.E. Rollin, S.F. Dowell, A.E. Ling, C.D. Humphrey, W.J. Shieh, J. Guarner, C.D. Paddock, P. Rota, B. Fields, J. DeRisi, J.Y. Yang, N. Cox, J.M. Hughes, J.W. LeDuc, W.J. Bellini, and L.J. Anderson. 2003. A novel coronavirus associated with severe acute respiratory syndrome. N. Engl. J. Med 348:1953-1966.
3. Peiris, J.S., S.T. Lai, L.L. Poon, Y. Guan, L.Y. Yam, W. Lim, J. Nicholls, W.K. Yee, W.W. Yan, M.T. Cheung, V.C. Cheng, K.H. Chan, D.N. Tsang, R.W. Yung, T.K. Ng, and K.Y. Yuen. 2003. Coronavirus as a possible cause of severe acute respiratory syndrome. Lancet 361:1319-1325.
4. Marra, M.A., S.J.M. Jones, C.R. Astell, R.A. Holt, A. Brooks-Wilson, Y.S.N. Butterfield, J. Khattra, J.K. Asano, S.A. Barber, S.Y. Chan, A. Cloutier, S.M. Coughlin, D. Freeman, N. Gim, O.L. Griffith, S.R. Leach, M. Mayo, H. McDonald, S.B. Montgomery, P.K. Pandoh, A.S. Petrescu, A.G. Robertson, J.E. Schein, A. Siddiqui, D.E. Smailus, J.M. Stott, G.S. Yang, F. Plummer, A. Andonov, H. Artsob, N. Bastien, K. Bernard, T.F. Booth, D. Bowness, M. Czub, M. Drebot, L. Fernando, R. Flick, M. Garbutt, M. Gray, A. Grolla, S. Jones, H. Feldmann, A. Meyers, A. Kabani, Y. Li, S. Normand, U. Stroher, G.A. Tipples, S. Tyler, R Vogrig, D. Ward, B. Watson, R.C. Brunham, M. Krajden, M. Petric, D.M. Skowronski, C. Upton, and R.L. Roper. 2003. The genome sequence of the SARS-associated coronavirus. Science 300:1399-1404.
5. Rota, P.A., M.S. Oberste, S.S. Monroe, W.A. Nix, R Campagnoli, J.P. Icenogle, S. Penaranda, B. Bankamp, K. Maher, M.H. Chen, S. Tong, A. Tamin, L. Lowe, M. Frace, J.L. DeRisi, Q. Chen, D. Wang, D.D Erdman, T.C.T. Peret, C. Burns, T.G. Ksiazek, P.E. Rollin, A. Sanchez, S. Liffick, B. Holloway, J. Limor, K. McCaustland, M. Olsen-Rasmussen, R. Fouchier, S. Gunther, A.D.M.E. Osterhaus, C. Drosten, M.A. Pallansch, L.J. Anderson, and W.J. Bellini. 2003. Characterization of a Novel Coronavirus Associated with Severe Acute Respiratory Syndrome. Science 300:1394-1399.
6. Fleck, F. 2004. SARS virus returns to China as scientists race to find effective vaccine. Bull. World Health Organ. 82:152-153.
7. Marshall, E. and M. Enserink. 2004. Medicine. Caution urged on SARS vaccines. Science 303:944-946.
8. Peiris, J.S., Y. Guan, and K.Y. Yuen. 2004. Severe acute respiratory syndrome. Nat. Med. 10:S88-597.
9. Qin, E., Q. Zhu, M. Yu, B. Fan, G. Chang, B. Si, B. Yang, W. Peng, T. Jiang, B. Liu, Y. Deng, H. Liu, Y. Zhang, C. Wang, Y. Li, Y. Gan, X. Li, F. Lu, G. Tan, W. Cao, R. Yang, J. Wang, W. Li, Z. Xu, Y. Li, Q. Wu, W. Lin, Y. Han, G. Li, W. Li, H. Lu, J. Shi, Z. Tong, F. Zhang, S. Li, B. Liu, S. Liu, W. Dong, J. Wang, G.K.S. Wong, J. Yu, and H. Yang. 2003. A complete sequence and comparative analysis of a SARS-associated virus (isolate BJ01). Chin. Sci. Bull. 48:941-948.
10. Holmes, K.V. and L. Enjuanes. 2003. Virology: The SARS coronavirus: a postgenomic era. Science 300:1377-1378.
11. Holmes, K.V. 2003. SARS coronavirus: a new challenge for prevention and therapy. J. Clin.. Invest 111:1605-1609.
12. Frana, M.F., J.N. Behnke, L.S. Sturman, and K.V. Holmes. 1985. Proteolytic cleavage of the E2 glycoprotein of murine coronavirus: host-dependent differences in proteolytic cleavage and cell fusion. J. Virol. 56:912-920.
13. Luytjes, W., L.S. Sturman, P.J. Bredenbeek, J. Charite, B.A. van der Zeijst, M.C. Horzinek, and W.J. Spaan. 1987. Primary structure of the glycoprotein E2 of coronavirus MHV-A59 and identification of the trypsin cleavage site. Virology 161:479-487.
14. Spiga, O., A. Bernini, A. Ciutti, S. Chiellini, N. Menciassi, F. Finetti, V. Causarono, F. Anselmi, F. Prischi, and N. Niccolai. 2003. Molecular modelling of S1 and S2 subunits of SARS coronavirus spike glycoprotein. Biochem. Biophys. Res. Commun. 310:84.
15. Bosch, B.J., B.E. Martina, Z.R Van Der, J. Lepault, B.J. Haijema, C. Versluis, A.J. Heck, R De Groot, A.D. Osterhaus, and P.J. Rottier. 2004. Severe acute respiratory syndrome coronavirus (SARS-CoV) infection inhibition using spike protein heptad repeat-derived peptides. Proc. Natl. Acad Sci. USA 101:8455-8460.
16. Ingallinella, P., E. Bianchi, M. Finotto, G. Cantoni, D.M. Eckert, V.M. Supekar, C. Bruckmann, A. Carfi, and A. Pessi. 2004. Structural characterization of the fusion-active complex of severe acute respiratory syndrome (SARS) coronavirus. Proc. Natl. Acad. Sci. USA 101:8709-8714.
17. Liu, S., G. Xiao, Y. Chen, Y. He, J. Niu, C. Escalante, H. Xiong, J. Farmar, A.K. Debnath, P. Tien, and S. Jiang. 2004. Interaction between the heptad repeat 1 and 2 regions in spike protein of SARS-associated coronavirus: implication for virus fusogenic mechanism and identification of fusion inhibitors. Lancet 363:938-947.
18. Tripet, B., M.W. Howard, M. Jobling, R.K. Holmes, K.V. Holmes, and R.S. Hodges. 2004. Structural characterization of the SARS-coronavirus spike S fusion protein core. J. Biol. Chem. 279:20836-20849.
19. Chan, D.C., D. Fass, J.M. Berger, and P.S. Kim. 1997. Core structure of gp41 from the HIV envelope glycoprotein. Cell 89:263-273.
20. Bosch, B.J., Z.R. van der, C.A. de Haan, and P.J. Rottier. 2003. The coronavirus spike protein is a class I virus fusion protein: structural and functional characterization of the fusion core complex. J. Virol. 77:8801-8811.
21. Xu, Y., Z. Lou, Y. Liu, H. Pang, P. Tien, G.F. Gao, and Z. Rao. 2004. Crystal structure of SARS-CoV spike protein fusion core. J. Biol. Chem. 279:49414-49419.
22. Li, W.H., M.J. Moore, N.Y. Vasilieva, J.H. Sui, S.K. Wong, A.M. Berne, M. Somasundaran, J.L. Sullivan, K. Luzuriaga, T.C. Greenough, H.Y. Choe, and M. Farzan. 2003. Angiotensin-converting enzyme 2 is a functional receptor for the SARS coronavirus. Nature 426:450-454.
23. Dimitrov, D.S. 2003. The secret life of ACE2 as a receptor for the SARS Virus. Cell 115:652-653.
24. Prabakaran, P., X. Xiao, and D.S. Dimitrov. 2004. A model of the ACE2 structure and function as a SARS-CoV receptor. Biochem. Biophys. Res. Commun. 314:235-241.
25. Wang, P., J. Chen, A. Zheng, Y. Nie, X. Shi, W. Wang, G. Wang, M. Luo, H. Liu, L. Tan, X. Song, Z. Wang, X. Yin, X. Qu, X. Wang, T. Qing, M. Ding, and H. Deng. 2004. Expression cloning of functional receptor used by SARS coronavirus. Biochem. Biophys. Res. Commun. 315:439-444.
26. Xiao, X., S. Chakraborti, A.S. Dimitrov, K. Gramatikoff, and D.S. Dimitrov. 2003. The SARS-CoV S glycoprotein: expression and functional characterization. Biochem. Biophys. Res. Commun. 312:1159-1164.
27. Wong, S.K., W. Li, M.J. Moore, H. Choe, and M. Farzan. 2004. A 193-amino-acid fragment of the SARS coronavirus S protein efficiently binds angiotensin-converting enzyme 2. J. Biol. Chem. 279:3197-3201.
28. Babcock, G.J., D.J. Esshaki, W.D. Thomas, Jr., and D.M. Ambrosino. 2004. Amino acids 270 to 510 of the severe acute respiratory syndrome coronavirus spike protein are required for interaction with receptor. J. Virol. 78:4552-4560.
29. Cavanagh,D. 1995. The coronavirus surface glycoprotein. In The Coronaviridae. S.G.Siddell, editor. Plenum Press, New York and London. 73-114.
30. Saif, L.J. 1993. Coronavirus immunogens. Vet. Microbiol. 37:285-297.
31. Buchholz, U.J., A. Bukreyev, L. Yang, E.W. Lamirande, B.R. Murphy, K. Subbarao, and P.L. Collins. 2004. Contributions of the structural proteins of severe acute respiratory syndrome coronavirus to protective immunity. Proc. Natl. Acad. Sci. USA 101:9804-9809.
32. Yang, Z.Y., W.P. Kong, Y. Huang, A. Roberts, B.R. Murphy, K. Subbarao, and G.J. Nabel. 2004. A DNA vaccine induces SARS coronavirus neutralization and protective immunity in mice. Nature 428:561-564.
33. Bisht, H., A. Roberts, L. Vogel, A. Bukreyev, P.L. Collins, B.R. Murphy, K. Subbarao, and B. Moss. 2004. Severe acute respiratory syndrome coronavirus spike protein expressed by attenuated vaccinia virus protectively immunizes mice. Proc. Natl. Acad. Sci. USA 101:6641-6646.
34. Bukreyev, A., E.W. Lamirande, U.J. Buchholz, L.N. Vogel, W.R. Elkins, M. St Claire, B.R. Murphy, K. Subbarao, and P.L. Collins. 2004. Mucosal immunisation of African green monkeys (Cercopithecus aethiops) with an attenuated parainfluenza virus expressing the SARS coronavirus spike protein for the prevention of SARS. Lancet 363:2122-2127.
35. He, Y., Y. Zhou, S. Liu, Z. Kou, W. Li, M. Farzan, and S. Jiang. 2004. Receptor-binding domain of SARS-CoV spike protein induces highly potent neutralizing antibodies: implication for developing subunit vaccine. Biochem. Biophys. Res. Commun. 324:773-781.
36. He, Y., Y. Zhou, P. Siddiqui, and S. Jiang. 2004. Inactivated SARS-CoV vaccine elicits high titers of spike protein-specific antibodies that block receptor binding and virus entry. Biochem. Biophys. Res. Commun. 325:445-452.
37. Morrison, S.L., M.J. Johnson, L.A. Herzenberg, and V.T. Oi. 1984. Chimeric human antibody molecules: mouse antigen-binding domains with human constant region domains. Proc. Natl. Acad Sci. USA 81:6851-6855.
38. Boulianne, G.L., N. Hozumi, and M.J. Shulman. 1984. Production of functional chimaeric mouse/human antibody. Nature 312:643-646.
39. Nisihara, T., Y. Ushio, H. Higuchi, N. Kayagaki, N. Yamaguchi, K. Soejima, S. Matsuo, H. Maeda, Y. Eda, K. Okumura, and H. Yagita. 2001. Humanization and epitope mapping of neutralizing anti-human Fas ligand monoclonal antibodies: structural insights into Fas/Fas ligand interaction. J. Immunol. 167:3266-3275.
40. Winter, G. and C. Milstein. 1991. Man-made antibodies. Nature 349:293-299.
41. Foote, J. and G. Winter. 1992. Antibody framework residues affecting the conformation of the hypervariable loops. J. Mol. Biol. 224:487-499.
42. Zhang, H., G. Wang, J. Li, Y. Nie, X. Shi, G. Lian, W. Wang, X. Yin, Y. Zhao, X. Qu, M. Ding, and H. Deng. 2004. Identification of an antigenic determinant on the S2 domain of the severe acute respiratory syndrome coronavirus spike glycoprotein capable of inducing neutralizing antibodies. J. Virol 78:6938-6945.
43. Nie, Y., G. Wang, X. Shi, H. Zhang, Y. Qiu, Z. He, W. Wang, G. Lian, X. Yin, L. Du, L. Ren, J. Wang, X. He, T. Li, H. Deng, and M. Ding. 2004. Neutralizing antibodies in patients with severe acute respiratory syndrome-associated coronavirus infection. J Infect. Dis. 190:1119-1126.
44. Hofmann, H., K. Hattermann, A. Marzi, T. Gramberg, M. Geier, M. Krumbiegel, S. Kuate, K. Uberla, M. Niedrig, and S. Pohlmann. 2004. S protein of severe acute respiratory syndrome-associated coronavirus mediates entry into hepatoma cell lines and is targeted by neutralizing antibodies in infected patients. J Virol 78:6134-6142.
45. Lu, L., I. Manopo, B.P. Leung, H.H. Chng, A.E. Ling, L.L. Chee, E.E. Ooi, S.W. Chan, and J. Kwang. 2004. Immunological characterization of the spike protein of the severe acute respiratory syndrome coronavirus. J Clin. Microbiol. 42:1570-1576.
46. He, Y., Y. Zhou, H. Wu, B. Luo, J. Chen, W. Li, and S. Jiang. 2004. Identification of immunodominant sites on the spike protein of severe acute respiratory syndrome (SARS) coronavirus: implication for developing SARS diagnostics and vaccines. J. Immunol. 173:4050-4057.
47. Wilson, J.A., M. Hevey, R. Bakken, S. Guest, M. Bray, A.L. Schmaljohn, and M.K. Hart. 2000. Epitopes involved in antibody-mediated protection from Ebola virus. Science 287:1664-1666.
48. Zwick, M.B., L.L. Bonnycastle, A. Menendez, M.B. Irving, C.F. Barbas, III, P.W. Parren, D.R. Burton, and J.K. Scott. 2001. Identification and characterization of a peptide that specifically binds the human, broadly neutralizing anti-human immunodeficiency virus type 1 antibody b12. J Virol 75:6692-6699.
49. Sui, J., W. Li, A. Murakami, A. Tamin, L.J. Matthews, S.K. Wong, MJ. Moore, A.S. Tallarico, M. Olurinde, H. Choe, L.J. Anderson, W.J. Bellini, M. Farzan, and W.A. Marasco. 2004. Potent neutralization of severe acute respiratory syndrome (SARS) coronavirus by a human mAb to S1 protein that blocks receptor association. Proc. Natl. Acad. Sci. USA 101:2536-2541.
50. Subbarao, K., J. McAuliffe, L. Vogel, G. Fahle, S. Fischer, K. Tatti, M. Packard, W.J. Shieh, S. Zaki, and B. Murphy. 2004. Prior infection and passive transfer of neutralizing antibody prevent replication of severe acute respiratory syndrome coronavirus in the respiratory tract of mice. J Virol 78:3572-3577.
51. ter Meulen, J., A.B.H. Bakker, E.N. van den Brink, G.J. Weverling, B.E.E. Martina, B.L. Haagmans, T. Kuiken, J. de Kruif, W. Preiser, W. Spaan, H.R. Gelderblom, J. Goudsmit, and A.D.M.E. Osterhaus. 2004. Human monoclonal antibody as prophylaxis for SARS coronavirus infection in ferrets. Lancet 363:2139-2141.
52. Traggiai, E., S. Becker, K. Subbarao, L. Kolesnikova, Y. Uematsu, M.R. Gismondo, B.R. Murphy, R. Rappuoli, and A. Lanzavecchia. 2004. An efficient method to make human monoclonal antibodies from memory B cells: potent neutralization of SARS coronavirus. Nat Med 10:871-875.
53. Welt, S., C.R. Divgi, F.X. Real, S.D. Yeh, P. Garin-Chesa, C.L. Finstad, J. Sakamoto, A. Cohen, E.R. Sigurdson, N. Kemeny, and 1990. Quantitative analysis of antibody localization in human metastatic colon cancer: a phase I study of monoclonal antibody A33. J. Clin Oncol. 8:1894-1906.
54. Welt, S. and G. Ritter. 1999. Antibodies in the therapy of colon cancer. Semin. Oncol. 26:683-690.
55. Breedveld, F.C. 2000. Therapeutic monoclonal antibodies. Lancet 355:735-740.
56. Xu, Z.K., L.X. Wei, L.Y. Wang, H.T. Wang, and S. Jiang. 2002. The In vitro and in vivo protective activity of monoclonal antibodies directed against Hantaan virus: potential application for immunotherapy and passive immunization. Biochem. Biophys. Res. Commun. 298:552-558.

The invention (not according to the invention) may be defined in accordance with aspects i to 36 below.
1. An isolated antibody capable of binding to the receptor-binding domain of the spike protein of the severe acute respiratory syndrome-associated coronavirus (SARS-CoV) or antibody capable of competitively inhibiting the binding of the severe acute respiratory syndrome-associated coronavirus (SARS-CoV) to a receptor on host cells or to a cell-free receptor.
2. A substance comprising the complementary determining regions of the antibody of aspect 1, capable of binding to the same epitope or competitively inhibiting the binding of said epitope as the antibody of aspect 1.
3. The substance of aspect 2, where the substance is an antibody.
4. The antibody of aspect 1, where the antibody is neutralizing.
5. An antibody produced by Hybridoma 18D9 with ATCC Accession No. PTA-6521.
6. The epitope recognized by the antibody of aspect 5.
7. An antibody produced by Hybridoma 19B2 with ATCC Accession No. PTA-6522.
8. The epitope recognized by the antibody of aspect 7.
9. An antibody produced by Hybridoma 30F9 with ATCC Accession No. PTA-6523.
10. The epitope recognized by the antibody of aspect 9.
11. An antibody produced by Hybridoma 31H12 with ATCC Accession No. PTA-6524.
12. The epitope recognized by the antibody of aspect 11.
13. An antibody produced by Hybridoma 32H5 with ATCC Accession No. PTA-6525.
14. The epitope recognized by the antibody of aspect 13.
15. An antibody produced by Hybridoma 33G4 with ATCC Accession No. PTA-6526.
16. The epitope recognized by the antibody of aspect 15.
17. The antibody of aspect 1, where the antibody is a single-chain antibody or an antibody-fusion construct.
18. The antibody of aspect 1, where the antibody is a humanized antibody.
19. The antibody of aspect 1, where the antibody is a chimeric antibody.
20. The isolated antibody of aspect 1, where the antibody is directly or indirectly coupled to a cytotoxic agent.
21. A cell comprising the antibody of aspect 1.
22. A nucleic acid molecule encoding the antibody of aspect 1.
23. A nucleic acid molecule capable of specifically hybridizing the molecule of aspect 22.
24. The nucleic acid molecule of aspect 22, wherein it is synthetic DNA, genomic DNA, cDNA, or RNA.
25. A vector comprising the nucleic acid molecule of aspect 24 or a portion thereof.
26. A cell comprising the nucleic acid molecule of aspect 24.
27. A method for producing an antibody capable of binding to the receptor-binding domain of the spike protein of the severe acute respiratory syndrome-associated coronavirus (SARS-CoV) or antibody capable of competitively inhibiting the binding of the severe acute respiratory syndrome-associated coronavirus (SARS-CoV) to host cells, comprising operatively-linking the nucleic acid molecule of aspect 22 to the appropriate regulatory element so as to express said antibody; placing the linked nucleic molecule in appropriate conditions permitting the expression of said antibody; and recovering said expressed antibody, thereby producing said antibody.
28. The antibody produced by the method of aspect 27.
29. A composition comprising an effective amount of the antibody of aspect 1 and a suitable carrier.
30. A pharmaceutical composition comprising an effective amount of the antibody of aspect 1 and a pharmaceutically-acceptable carrier.
31. A method for treating infection of severe acute respiratory syndrome-associated coronavirus (SARS-CoV), comprising the use of the pharmaceutical composition of aspect 30.
32. A method of preventing infection of severe acute respiratory syndrome-associated coronavirus (SARS-CoV), comprising the use of the pharmaceutical composition of aspect 30.
33. A method for detecting severe acute respiratory syndrome-associated coronavirus (SARS-CoV) or the SARS-Co V-infected cells, comprising contacting the antibody or its derivative capable of binding to the receptor-binding domain of the spike protein of said virus under conditions permitting the formation of complexes between the antibody, or its derivative, and the receptor-binding domain of spike protein of the severe acute respiratory syndrome-associated coronavirus (SARS-CoV); and detecting the complexes formed.
34. A method for screening compounds capable of inhibiting infection of severe respiratory syndrome-associated coronavirus by blocking the binding of said virus to receptors on host cells, comprising the steps of:
   (a) establishing a system for the antibody of aspect 1 to bind to the receptor-binding domain of the spike protein of the severe acute respiratory syndrome-associated coronavirus (SARS-CoV); and
   (b) contacting the compounds with the system of (a), a decrease in binding of the antibody of aspect 1 to the receptor-binding domain of the spike protein of the severe acute respiratory syndrome-associated coronavirus (SARS-CoV) indicating that the compounds are capable of interfering with said binding and inhibiting infection of the receptor-binding domain of the spike protein of the severe acute respiratory syndrome-associated coronavirus (SARS-CoV).
35. The compound resulting from the method of aspect 34.
36. A kit comprising a compartment containing the antibody of aspect 1.

## Claims

1. An isolated neutralising monoclonal antibody capable of binding to the receptor-binding domain of the spike protein of the severe acute respiratory syndrome-associated coronavirus (SARS-CoV) wherein the antibody is produced by Hybridoma 30F9 with ATCC Accession No. PTA-6523.

2. A pharmaceutical composition comprising an effective amount of the antibody according to claim 1 and a pharmaceutically-acceptable carrier.

3. Hybridoma 30F9 with ATCC Accession No. PTA-6523.

4. An antibody according to claim 1 for use in the treatment of SARS-CoV infection.

5. Use of an antibody according to claim 1 in the preparation of a medicament for the treatment of SARS-CoV infection.

6. A method for detecting severe acute respiratory syndrome-associated coronavirus (SARS-CoV) or SARS-CoV-infected cells *in vitro,* comprising contacting an antibody according to claim 1 under conditions permitting the formation of complexes between the antibody and the receptor-binding domain of the spike protein of the SARS-CoV, and detecting the complexes formed.

7. A method for screening compounds capable of inhibiting infection by severe respiratory syndrome-associated coronavirus (SARS-CoV) by blocking the binding of the virus to receptors on host cells, comprising the steps of:
(a) establishing a system for an antibody according to claim 1 to bind to the receptor-binding domain of the spike protein of the SARS-CoV; and
(b) contacting a compound to be screened with the system of (a), a decrease in binding of the antibody to the receptor-binding domain of the spike protein of the SARS-CoV indicating that the compound is capable of interfering with the binding and inhibiting infection of the receptor-binding domain of the spike protein of SARS-CoV.

8. A diagnostic kit or a screening kit comprising a compartment containing an antibody according to claim 1.

## Patentansprüche

1. Isolierter neutralisierender monoklonaler Antikörper, der an die Rezeptorbindungsdomäne des Spike-Proteins des mit dem schweren akuten Atemwegssyndrom assoziierten Coronavirus (severe acute respiratory syndrome-associated coronavirus, SARS-CoV) binden kann, wobei der Antikörper von dem Hybridom 30F9 mit der ATCC-Zugangsnr. PTA-6523 produziert wird.

2. Pharmazeutische Zusammensetzung, die eine wirksame Menge des Antikörpers nach Anspruch 1 und einen pharmazeutisch unbedenklichen Trägerstoff umfasst.

3. Hybridom 30F9 mit der ATCC-Zugangsnr. PTA-6523.

4. Antikörper nach Anspruch 1 zur Verwendung bei der Behandlung einer Infektion mit SARS-CoV.

5. Verwendung eines Antikörpers nach Anspruch 1 bei der Herstellung eines Arzneimittels zur Behandlung einer Infektion mit SARS-CoV.

6. Verfahren zum Nachweisen von mit dem schweren akuten Atemwegssyndrom assoziiertem Coronavirus (SARS-CoV) oder mit SARS-CoV infizierten Zellen *in vitro,* wobei das Verfahren das Inkontaktbringen mit einem Antikörper nach Anspruch 1 unter Bedingungen, die die Bildung von Komplexen zwischen dem Antikörper und der Rezeptorbindungsdomäne des Spike-Proteins des SARS-CoV ermöglichen, und das Nachweisen der gebildeten Komplexe umfasst.

7. Verfahren zum Screenen von Verbindungen, die eine Infektion mit dem mit dem schweren akuten Atemwegssyndrom assoziierten Coronavirus (SARS-CoV) hemmen können, indem sie die Bindung des Virus an Rezeptoren an Wirtszellen blockieren, wobei das Verfahren die folgenden Schritte umfasst:
(a) Herstellen eines Systems für einen Antikörper nach Anspruch 1 zum Binden an die Rezeptorbindungsdomäne des Spike-Proteins des SARS-CoV und
(b) Inkontaktbringen einer zu screenenden Verbindung mit dem System von (a), wobei eine Verringerung der Bindung des Antikörpers an die Rezeptorbindungsdomäne des Spike-Proteins des SARS-CoV anzeigt, dass die Verbindung die Bindung stören und eine Infektion der Rezeptorbindungsdomäne des Spike-Proteins von SARS-CoV hemmen kann.

8. Diagnosekit oder Screening-Kit, das eine Kammer umfasst, die einen Antikörper nach Anspruch 1 enthält.

## Revendications

1. Anticorps monoclonal neutralisant isolé capable de se lier au domaine de liaison du récepteur à la protéine S du coronavirus associé au syndrome respiratoire aigu sévère (SARS-CoV), où l'anticorps est produit par l'hybridome 30F9 déposé sous le numéro ATCC PTA-6523.

2. Composition pharmaceutique comprenant une quantité efficace de l'anticorps selon la revendication 1 et un véhicule pharmaceutiquement acceptable.

3. Hybridome 30F9 déposé sous le numéro ATCC PTA-6523.

4. Anticorps selon la revendication 1, en vue d'une utilisation dans le traitement d'une infection par le SARS-CoV.

5. Utilisation d'un anticorps selon la revendication 1 dans la préparation d'un médicament pour le traitement d'une infection par le SARS-CoV.

6. Méthode de détection du coronavirus associé au syndrome respiratoire aigu sévère (SARS-CoV) ou de cellules infectées par le SARS-CoV *in vitro*, comprenant la mise en contact d'un anticorps selon la revendication 1 dans des conditions permettant la formation de complexes entre l'anticorps et le domaine de liaison du récepteur à la protéine S du SARS-CoV et la détection des complexes formés.

7. Méthode de triage de composés capables d'inhiber une infection par le coronavirus associé au syndrome respiratoire aigu sévère (SARS-CoV) en bloquant la liaison du virus aux récepteurs sur les cellules hôtes, la dite méthode comprenant les étapes consistant à :
(a) établir un système pour qu'un anticorps selon la revendication 1 se lie au domaine de liaison du récepteur à la protéine S du SARS-CoV ; et
(b) mettre en contact le composé à tester avec le système établi en (a), une diminution de la liaison de l'anticorps au domaine de liaison du récepteur à la protéine S du SARS-CoV indiquant que ledit composé est capable d'interférer sur la liaison au domaine de liaison du récepteur à la protéine S du SARS-CoV et d'inhiber ainsi l'infection.

8. Kit de diagnostic ou de dépistage comprenant un compartiment qui contient un anticorps selon la revendication 1.
